(19) Europäisches Patentamt
European Patent Office
Office européen des brevets

(11) Veröffentlichungsnummer: **0 008 804**
**B1**

(12) # EUROPÄISCHE PATENTSCHRIFT

(45) Veröffentlichungstag der Patentschrift: **16.11.83**

(21) Anmeldenummer: **79103360.8**

(22) Anmeldetag: **07.09.79**

(51) Int. Cl.³: **C 07 D 233/60,**
C 07 D 233/91,
C 07 D 233/68,
A 61 K 31/415,
A 01 N 43/50

(54) **Imidazolylvinylether, Verfahren zu deren Herstellung, deren Verwendung und diese Ether enthaltende Arzneimittel und Pflanzenschutzmittel.**

(30) Priorität: **11.09.78 DE 2839388**

(43) Veröffentlichungstag der Anmeldung:
**19.03.80 Patentblatt 80/6**

(45) Bekanntmachung des Hinweises auf die Patenterteilung:
**16.11.83 Patentblatt 83/46**

(84) Benannte Vertragsstaaten:
**AT BE CH DE FR GB IT LU NL SE**

(56) Entgegenhaltungen:
**DE - A - 2 645 617**
**DE - A - 2 757 113**

(73) Patentinhaber: **SIEGFRIED AKTIENGESELLSCHAFT**
**CH-4800 Zofingen (CH)**

(72) Erfinder: **Zirngibl, Ludwig, Dr.**
**Eisengrubenweg 16**
**CH-4800 Zofingen (CH)**
Erfinder: **Fischer, Johanna, Dr.**
**Mattenstrasse 10**
**CH-6260 Reiden (CH)**
Erfinder: **Thiele, Kurt, Dr.**
**Rebbergstrasse 47d**
**CH-4800 Zofingen (CH)**

(74) Vertreter: **Jaeger, Klaus, Dr.rer.nat. Dipl.-Chem. et al,**
**Jaeger, Grams & Pontani Patentanwälte**
**Bergstrasse 48 1/2**
**D-8035 München-Gauting (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 008 804 B1

Imidazolylvinylether, Verfahren zu deren Herstellung, deren Verwendung und diese Ether enthaltende Arzneimittel und Pflanzenschutzmittel

Die Erfindung betrifft Imidazolylvinylether der Formel I

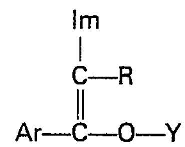

(I)

und deren Säureadditionssalze, wobei die Reste Ar, R, Im und Y die im Patentanspruch 1 genannte Bedeutung haben. Die Erfindung betrifft weiterhin ein Verfahren zur Herstellung dieser Imidazolylvinylether sowie humanmedizinische oder veterinärmedizinische Arzneimittel und Pflanzenschutzmittel, die diese Imidazolylvinylether und deren für den jeweiligen Einsatzzweck unbedenklichen Säureadditionssalze als fungiziden und/oder bakteriziden Wirkstoff enthalten.

Aus der Druckschrift DE—A—26 45 617 sind Phenyl-vinyl-ketone bekannt, die am $\alpha$-Vinylkohlenstoffatom eine Imidazol-1-yl-gruppe tragen. Diese Imidazolylvinylderivate können am $\beta$-Vinylkohlenstoffatom gegebenenfalls substituierte, insbesondere mit 1 oder 2 Chloratomen substituierte Phenylreste tragen. Diese Phenyl-imidazolylvinylketone sind fungizide Wirkstoffe ohne bemerkenswerte bakterizide Eigenschaften.

Außerdem ist $\alpha$-(2,4-Dichlorphenyl)-$\beta$-imidazol-1-yl-ethyl-(4-chlorphenyl)-methylethernitrat der Wirkstoff eines anerkannt gut wirksamen, im Handel erhältlichen Antimykotikums. Neben der antimykotischen Aktivität zeigt dieser Wirkstoff auch eine gewisse bakterizide Aktivität, die jedoch stark selektiv ist und beispielsweise gegen Streptococcus faecalis nicht gegeben ist.

Die vorstehend gegannten antimykotisch aktiven Wirkstoffe weisen weiterhin den Nachteil auf, daß sie gegenüber einer Reihe von Pilzen, aber auch Bakterien, bereits nach kurzer Zeit Resistenzen hervorrufen, und zwar insbesondere gegenüber Candida albicans.

Angesichts dieses Standes der Technik liegt der Erfindung die Aufgabe zugrunde, auch bakterizid wirkende antimykotisch aktive Wirkstoffe zu schaffen, die bei zumindest im wesentlichen vergleichbarer oder besserer antimykotischer und bakterizider Aktivität als Alternative eingesetzt werden können, wenn bei Verwendung der bekannten Antimykotika Resistenzen auftreten, bzw. die bekannten Antimykotika nicht ansprechen.

Zur Lösung dieser Aufgabe schafft die Erfindung die im Anspruch 1 spezifizierten neuen Imidazolylvinylether. Hergestellt werden die Imidazolylvinylether der Formel I durch Umsetzen eines entsprechenden Ketons der Formel III

$$Ar—CO—CHR—Im \qquad (III),$$

in der Ar, R und Im die im Patenanspruch I genannte Bedeutung haben, in Gegenwart von Alkalimetallen, Erdalkalimetallen, deren Hydriden oder Alkoholaten, lithiumorganischen Verbindungen, Natriumamid oder einfach oder zweifach N-substituierten Natriumamiden in einem Lösungsmittel mit einer Verbindung der Formel IV

$$Z—Y \qquad (IV),$$

in der Z eine alkalisch abspaltbare Gruppe, insbesondere ein Halogenatom ist.

Die Umsetzung erfolgt vorzugsweise in Gegenwart von NaH unter anfänglicher Eiskühlung und anschliessender milder Erwärmung. Dabei wird die Umsetzung insbesondere unter Rühren in einem Lösunsgsmittel durchgeführt, und zwar vorzugsweise in Hexamethylphosphorsäuretriamid.

In einigen wenigen Fällen lässt sich bei der vorstehend beschriebenen Art der Herstellung eine C-Alkylierung nicht immer ganz ausschliessen. Die dabei als Nebenprodukte erhaltenen Ethanonderivate der allgemeinen Formel V

$$Ar—CO—CRY—Im \qquad (V)$$

werden bei der säulenchromatographischen Trennung erst deutlich nach den weniger polaren Substanzen der Allgemeinen Formel I eluiert, sind also ohne weiteres von den Zielprodukten abtrennbar.

Neben dem vorstehend genannten Natriumhydrid, das vorzugsweise in öliger Dispersion eingesetzt wird, können als Kondensationsmittel auch Alkalimetalle und Erdalkalimetalle sowie deren Hydride und Alkoholate, lithiumorganische Verbindungen, Natriumamid oder einfach oder zweifach N-substituierte Natriumamide eingesetzt werden.

In der Formel I ist Ar vorzugsweise Phenyl oder halogensubstituiertes Phenyl, insbesondere Dichlorphenyl, und zwar speziell 2,4-Dichlorphenyl. Dabei hat r vorzugsweise die Bedeutung Wasserstoff oder Methyl. Y ist in diesem Zusammenhang vorzugsweise ein gegebenenfalls durch die Brückengruppen —O—, —S—, —SO— oder —$SO_2$— unterbrochener Alkylrest mit 2 bis 4 Kohlenstoffatomen, der endständig eine Arylgruppe mit der im Patentanspruch 1 definierten Bedeutung für Ar tragen kann. Die Imidazol-1-yl-Gruppe ist dabei vorzugsweise unsubstituiert.

Die Erfindung ist im folgendem anhand der Beispiele näher erläutert.

Beispiel 1

1-(2,4-Dichlorphenyl)-1-(methoxymethoxy)-2-(imidazol-1-yl)propen

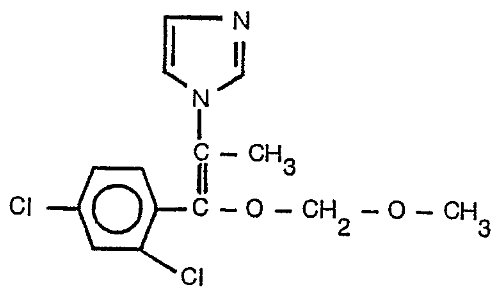

6,73 g (25 mmol) 2,4-Dichlor-$\alpha$-(imidazol-1-yl)propiophenon (J.med.Chem. *12*, 790) werden im 30 ml Hexamethylphosphorsäuretriamid gelöst und im Verlaufe vom 1,5 h bei 5°C mit 0,63 g (26 mmol) einer 50 %-igen NaH-Dispersion in Öl versetzt. Das Gemisch wird 1 h auf 50°C erwärmt, anschließend auf 5°C abgekühlt und tropfenweise mit 2,26 g (28 mmol) Chlordimethyläther versetzt. Dabei wird die Temperatur im Reaktionsgemisch durch Kühlen auf 5 bis 10°C gehalten. Nach der Zugabe des Chlordimethyläthers wird 1 h bei Raumtemperatur und 5 h bei 50°C gerührt. Anschließend wird das Reaktionsgemisch in Wasser eingegossen. Das sich abscheidende Öl wird in chloroform aufgenommen. Nach dem Abtrennen der organischen Phase wird die wässrige Phase noch mit Äther extrahiert. Die Extrakte werden getrennt getrocknet, vereinigt und vollständig eingedampft. Es werden 5,27 g einer öligen Substanz erhalten, die unter Verwendung von Chloroform als laufmittel zweimal über eine Kieselgelsäule chromatographiert werden. Auf diese Weise werden schließlich 1,66 g des reinen Endprodukts als ölige Substanz erhalten.

Die Reinheit der erhalten substanz wird im IR-Spektrum überprüft.

Elementaranalyse für $C_{14}H_{14}Cl_2N_2O_2$ (MG 313,2):

| | C(%) | H(%) | N(%) | Cl(%) |
|---|---|---|---|---|
| berechnet | 53,69 | 4,51 | 8,94 | 22,64 |
| gefunden | 52,45 | 4,91 | 8,83 | 22,87 |

Beispiel 2

1-(2,4-Dichlorphenyl)-1-(4-chlorphenoxymethoxy)-2-(imidazol-1-yl)propennitrat

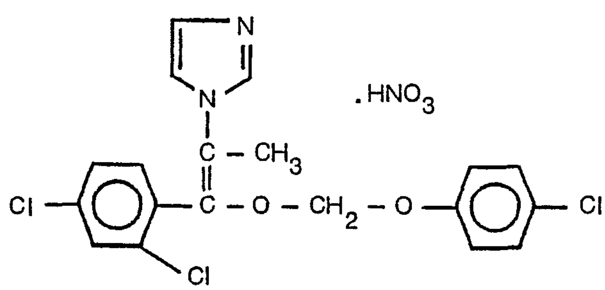

Das im Beispiel 1 beschriebene Verfahren wird mit der Abänderung wiederholt, daß statt des Chlordimethyläthers die äquivalente Menge Chlormethyl-4-chlorphenyläther eingesetzt wird. Nach dem Umkristallisieren weist das reine kristalline Nitrat einen Schmelzpunkt von 115,5 bis 118,5°C auf.

Elementaranalyse für $C_{19}H_{15}Cl_3N_2O_2 \cdot HNO_3$ (MG 472,7):

| | C(%) | H(%) | N(%) | O(%) |
|---|---|---|---|---|
| berechnet | 48,27 | 3,42 | 8,88 | 16,92 |
| gefunden | 48,21 | 3,37 | 8,59 | 16,88 |

Die $^1H$—NMR-Spektren ergeben bei 100 MHz in $d_6$-DMSO folgende Signallagen $\delta$(ppm): 1,90 (s, 3h,

—$CH_3$); 5,23 (s, 2H, —$OCH_2O$—), 6,84 (d, 2H, aromat. H (2') und (6')); 7,25 (d, 2H, aromat. H (3') und (5')); 7,63—7,82 (m, 5H, aromat. H und imidazolyl-4,5-H); 9,24 (s, 1H, imidazolyl-2H).

Beispiel 3

2-(2,4-Dichlorphenyl-2-(ethoxy)-1-(imidazol-1-yl)ethylennitrat

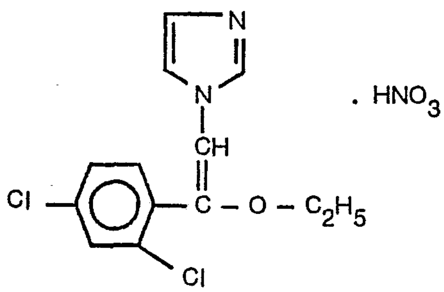

In einem Dreihalskolben mit Rückflußkühler, Tropftrichter und Rührmagneten werden 5,10 g (20 mmol) 1-(2,4-Chlorphenacyl)imidazol (J.med.Chem. *12* 790) in 25 ml Hexamethylphosphorsäuretriamid gelöst. Die Lösung wird mit 0,96 g (20 mmol) NaH in Form einer 50%-igen NaH-Dispersion in Öl versetzt. Es wird 2 h bei Raumtemperature und anschließend1 h bei 45°C gerührt. Nach dem Abkühlen werden 1,62 ml (20 mmol) Ethyliodid zugetropft. Die Reaktion verläuft stark exotherm. Unter Eiskühlung wird während der Zugabe des Ethyliodids im Reaktionsgemisch eine Temperatur von 10 bis 15°C eingehalten. Anschließend wird 20 h bei Raumtemperatur gerührt. Das Reaktionsgemisch wird dann in 300 ml Wasser eingegossen und dreimal mit Essigester ausgeschüttelt. Die vereinigten organischen Phasen werden mit Natriumsulfat getrocknet und vollständig eingedampft. Dabei werden 8,7 g einer öligen Substanz erhalten, die unter Verwendung von Dichlormethan als Laufmittel auf einer Kieselgelsäule chromatographisch gereinigt werden. Die Reinheit der Fraktionen wird im Dünnschichtchromatogramm überprüft. Die Eluatfraktionen mit gleichem und reinem Dünnschichtchromatogramm werden vereinigt, eingedampft, in Essigester aufgenommen und mit $HNO_3$ gefällt. Es werden 1,9 g reines Nitrat mit einem Schmelzpunkt von 131 bis 134°C erhalten.
Elementaranalyse für $C_{13}H_{12}Cl_2N_2O{\cdot}HNO_3$ (MG 356,2):

| | C(%) | H(%) | N(%) |
|---|---|---|---|
| berechnet | 45,10 | 3,78 | 12,14 |
| gefunden | 45,30 | 3,74 | 12,00 |

Im $^1H$—NMR-Spektrum werden bei 100 MHz in $d_6$-DMSO folgende Signallagen in $\delta$(ppm) beobachtet: 1,22 (t, 3H, —$CH_3$); 3,74 (q, 2H, —$CH_2$—); 6,75 (s, 1H, C=CH—); 7,60 (s, 2H, imidazolyl-4,5-H); 7,78 (d, 2H, aromat. H (5), H (6)); 7,98 (s, 1H, aromat. H (3)); 9,38 (s, 1H, imidazolyl-2-H).

Beispiel 4

2-(2,4-Dichlorphenyl)-2-(2-(4-chlorphenoxy)-ethoxy))-1-(imidazol-1-yl)ethylennitrat

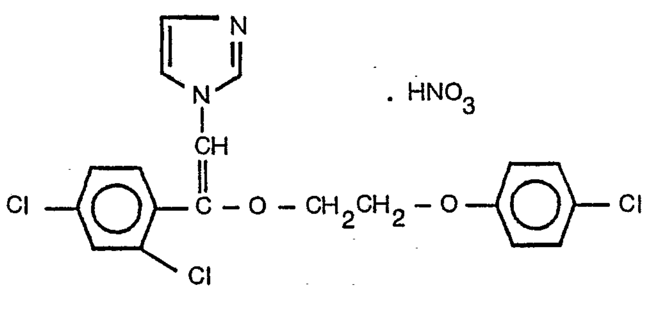

Das im Beispiel 3 beschriebene Verfahren wird mit der Abänderung wiederholt, daß statt des Ethyliodids eine äquivalente Menge 4-Chlorphenyl-2-iodethyläther eingesetzt wird. Das in Form weißer Kristalle anfallende Nitrat hat einen Schmelzpunkt von 151 bis 153°C. Bei Verwendung von 4-Chlorphenyl-2-bromethylether wird das gleiche Produkt erhalten.

Beispiel 5

2-(4-Chlorphenoxyethoxyethoxy)-2-(2,4-dichlorphenyl)-1-(imidazol-1-yl)ethylennitrat

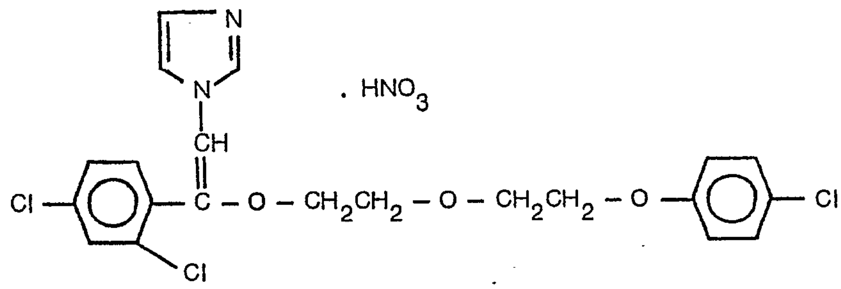

2,2'-Dichlordiethyläther und p-Chlorphenol werden in äquivalenten Mengen in verdünnter wässriger Natronlauge zu 4-Chlorphenoxyethoxy-ethylchlorid umgesetzt und in üblicher Weise aufgearbeitet. Das erhaltene reine 4-Chlorphenoxyethoxy-ethylchlorid hat unter einem Druck von 0,0013 mbar einen Siedepunkt von 110 bis 113°C
($n_{20}^{D}$ 1,5315: $d_{20}^{20}$ 1,249).

Analog zu dem in Beispiel 3 beschriebenen Verfahren werden 20 mmol des so erhaltenen 4-Chlorphenoxyethoxy-ethylchlorids mit der äquivalenten Menge 1-(2,4-Dichlorphenacyl)imidazol umgesetzt.

Das schließlich erhaltene gereinigte Nitrat hat einen Schmelzpunkt von 92 bis 94°C und zeigt im IR-Spektrum (in KBr) keine Carbonylbande.

Beispiel 6

2-(2,4-Dichlorphenyl)-2-(3-(4-chlorphenoxy)-propoxy)-1-(imidazol-1-yl)ethylennitrat

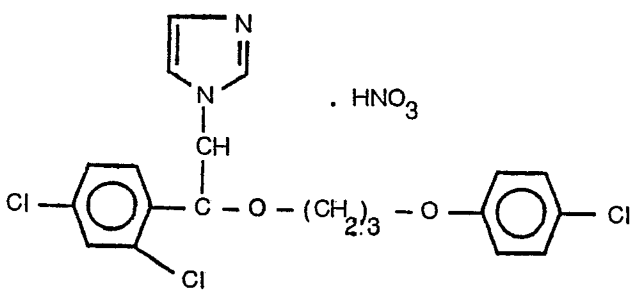

Das analog zu dem im Beispiel 3 ausführlich beschriebenen Verfahren hergestellte Nitrat hat einen Schmelzpunkt von 116 bis 117°C und weist im IR-Spektrum (in KBr) keine Carbonylbande mehr auf.

Analog zu der im Beispiel 1 beschriebenen Verfahrensweise werden die folgenden Verbindungen hergestellt:
1-(2,4-Dichlorphenyl)-1-(n-butoxy)-2-(imidazol-1-yl)propen;
1-(2,4-Dichlorphenyl)-1-(n-hexyloxy)-2-(imidazol-1-yl)propen; und
1-(2,4-Dichlorphenyl)-1-(N,N-diethylaminoethoxy)-2-(imidazol-1-yl)propen.

Beispiel 7

2-(2,4-Dichlorphenyl)-2-(2-(4-chlorphenoxy)-ethoxy)-1-(imidazol-1-yl)ethylennitrat

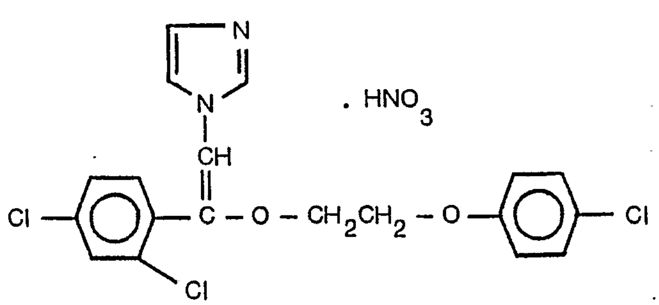

Die bereits nach Beispiel 4 hergestellte Substanz wird nach einer zweiten Verfahrensvariante wie folgt hergestellt:

Ein Dreihalskolben mit Rückflußkühler, Tropftrichter und Magnetrührer wird mit einer Lösung von 16,14 g (63 mmol) 1-(2,4-Dichlorphenacyl)imdazol in 90 ml Hexamethylphosphorsäuretriamid beschickt. Die Lösung wird in 2 Portionen mit insgesamt 2,88 g (ca. 69 mmol) Natriumhydrid in Form

einer 55 bis 60 gew.-%-igen öligen Dispersion versetzt. Es wird 1 h bei Raumtemperatur und anschließend 20 min bei 60°C gerührt. Nach Abkühlen auf Zimmertemperatur werden 14,13 g (60 mmol) 2-(4-Chlorphenoxy)ethylbromid zugetropft, das aus 1,2-Dibromethan hergestellt worden ist (Bull.Soc.-Chim. France *1957*, 1014). Die Zugabe erfolgt unter Rühren. Anschließend wird weitere 2 h bei Raumtemperatur gerührt. Das Reaktionsgemisch wird 14 bis 16 h bei Raumtemperatur stehengelassen und anschließend noch einmal 1 h auf 60°C erwärmt. Nach dem Abkühlen wird das Reaktionsgemisch in 1,6 1 Wasser eingegossen un mit Essigester extrahiert. Die vereinigten Extrakte werden mit Natriumsulfat getrocknet. Anschließend wird das Lösungsmittel unter vermindertem Druck abgezogen. Als Rückstand werden 29,2 g eines Öls erhalten, aus dem mit konzentrierter Salpetersäure unter Zusatz von Äther 9,2 g (19,5 mmol) des Produktnitrats gefällt werden. Nach Umkristallisieren aus 50 %-igem wässrigen Alkohol wird das kristalline Produkt mit einem Schmelzpunkt von 151 bis 153°C erhalten. Das IR-Spektrum dieser Substanz zeigt keine Carbonylbande mehr.
Elementaranalyse für $C_{19}H_{15}Cl_3N_2O_2 \cdot HNO_3$ (MG 472,2):

| | C(%) | H(%) | N(%) |
|---|---|---|---|
| berechnet | 48,27 | 3,41 | 8,89 |
| gefunden | 48,57 | 3,35 | 9,35 |

Beispiel 8

2-(2,4-Dichlorphenyl)-2-(n-butoxy)-1-(imidazol-1-yl)ethylennitrat

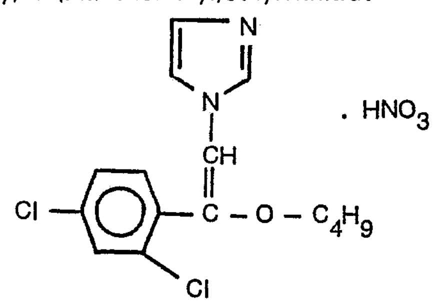

Die Substanz wird analog zu dem im Beispiel 7 beschriebenen Verfahren hergestellt. Nach dem Umkristallisieren aus wässrigem Alkohol schmilzt das weiße kristalline Produkt bei 153 bis 154°C.

Beispiel 9

2-(2,4-Dichlorphenyl)-2-(n-hexyloxy)-1-(imidazol-1-yl)-ethylennitrat

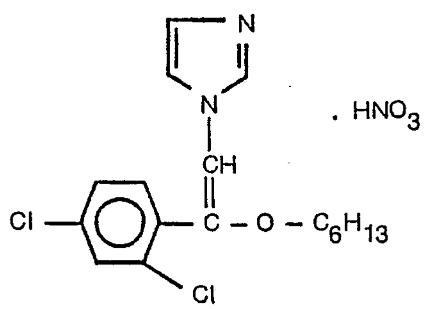

Die Substanz wird analog zu der im Beispiel 7 beschriebenen Weise hergestellt. Nach dem Umkristallisieren zeigt das gereinigte Nitrat einen Schmelzpunkt von 113 bis 115°C.

Beispiel 10

2-(2,4-Dichlorphenyl)-2-(2-diethylaminoethyloxy)-1-(imidazol-1-yl)ethylendinitrat

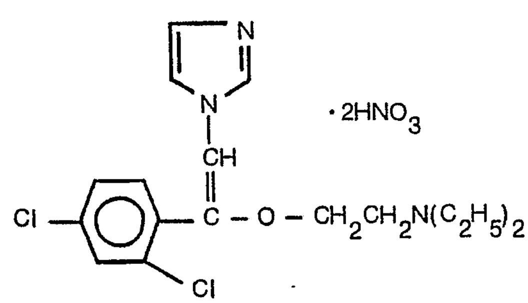

Die Substanz wird analog zu der im Beispiel 7 beschriebenen Verfahrensweise hergestellt. Nach Ansäuren mit konzentrierter Salpetersäure fällt im Gegensatz su den anderen Imidazolylvinyläthern nicht das Mononitrat, sondern das Dinitrat in kristalliner Form aus. Das Dinitrat hat nach dem Umkristallisieren einen Schmelzpunkt von 117 bis 118.5°C.

Beispiel 11

1-(2,4-Dichlorphenyl)-1-(4-chlorphenoxyethoxy)-2-(imidazol-1-yl)propennitrat

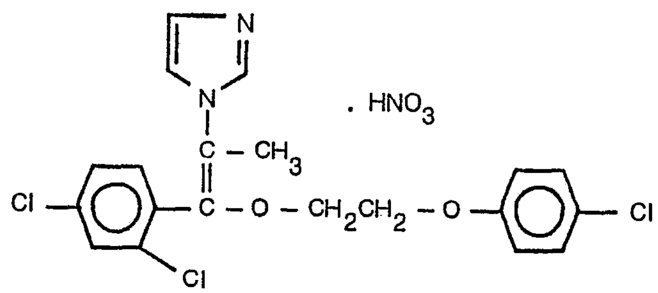

Die Substanz wird analog zu der im Beispiel 1 beschriebenen Weise mit anschließender Fällung mit konzentrierter Salptersäure und Kristallisieren aus wässrigem Alkohol hergestellt. Das erhaltene kristalline Produktnitrat hat einen Schmelzpunkt von 115 bis 123°C

Beispiele 12 bis 16

Wie insbesondere im Beispiel 1 beschrieben, werden die folgenden Substanzen hergestellt:

Beispiel 12: 2-(1-Naphthyl)-2-(n-butoxyethoxy)-1-(imidazol-1-yl)ethylennitrat.

Beispiel 13: 2-(2-Thienyl)-2-(2-(4-chlorphenoxy)-ethoxy)-1-(imidazol-1-yl)ethylennitrat mit dem Schmelzpunkt 172 bis 174°C.

Beispiel 14: 2-(4-Nitrophenyl)-2-n-butoxy-1-(2-methylimidazol-1-yl)ethylennitrat vom Schmelzpunkt 119 bis 120°C.

Beispiel 15: 1-(2,4-Dichlorphenyl)-1-(2-(4-chlorphenoxy)ethoxy)-2-(imidazol-1-yl)butennitrat, wobei das eine Isomer einen Schmelzpunkt von 106 bis 109°C, das andere einen Schmelzpunkt von 86 bis 87°C aufweist.

Beispiel 16: 2-(2,4-Dichlorphenyl)-2-(2-(4-chlorbenzyloxy)ethoxy)-1-(imidazol-1-yl)ethylennitrat vom Schmelzpunkt 119 bis 121°C.

Versuche:

Die nach den Beispielen 1 bis 11 hergestellten Substanzen werden auf ihre bakterizide und fungizide Aktivität geprüft. Zum Vergleich wird unter identischen Bedingungen das im Handel erhältliche anerkannte Antimykotikum $\alpha$-(2,4-Dichlorphenyl)-$\beta$-imidazol-1-yl-ethyl-(4-chlorphenyl)-methyläthernitrat, in der Tabelle 1 mit "A" bezeichnet, geprüft.

Zur Bestimmung der minimalen Hemmkonzentration (MIC) wird die Gradientenplattenmethode mit Gradienten von Null bis 100 $\mu$g/ml herangezogen. Die zu prüfenden Substanzen werden als Lösungen in 10%-igem Dimethylformamid eingesetzt. Die Ablesung der Ergebnisse erfolgt drei Tage nach Versuchsbeginn. Die erhaltenen Daten sind in der Tabelle 1 zusammengestellt. Als Testorganismen dienen die Bakterien Staphyloccoccus aureus haemolyticus (St) und Streptococcus faecalis (Str) und die Pilze Candida albicans (Ca), Trichophyton mentagrophytes (Tri) und Aspergillus niger (Asp). Die Fälle, in denen Resistenzen oder Teilresistenzen beobachlet werden, sind in der Tabelle 1 durch "r" gekennzeichnet.

Tabelle 1
MIC (µg/ml)

| Beispiel Nr. | Bakterien | | Pilze | | |
|---|---|---|---|---|---|
| | St | Str | Ca | Tri | Asp |
| 1 | 60 | 60 | <10 | <10 | <10 |
| 2 | <10 | <10 | r | <10 | <10 |
| 3 | 25 | 15 | <10 | <10 | <10 |
| 4 u.7 | <10 | r | r | <10 | <10 |
| 5 | 10 | 60r | r | <10 | 50r |
| 6 | 10 | 40r | — | <10 | 20r |
| 8 | <10 | <10 | <10 | <10 | <10 |
| 9 | <10 | <10 | r | <10 | <10 |
| 11 | <10 | <10 | r | <10 | <10 |
| A | <10 | — | r | <10 | <10 |

Beispiele 17 bis 28

Analog zu den in den vorstehenden Beispielen beschriebenen Verfahren werden die folgenden Substanzen hergestellt und in der ebenfalls vorstehend beschriebenen Weise mikrobiologisch hinsichtlich ihrer Wirksamkeit gegenüber Bakterien und Pilzen geprüft.

Beispiel 17: 1-(2,4-Dichlorphenyl)-1-(n-octyloxy)-2-(imidazol-1-yl)ethylennitrat vom Schmelzpunkt 96 bis 98°C.

Beispiel 18: 2-(4-Chlorphenyl)-2-[2-(2,4-dichlorphenoxy)-ethoxy]-1-(imidazol-1-yl)ethylennitrat vom Schmelzpunkt 122 bis 124°C.

Beispiel 19: 2-(2,4-Dichlorphenyl)-2-[2-(4-chlorphenoxy)-ethoxy]-(2-ethyl-imidazol-1-yl)ethylen, das als Öl anfällt.

Beispiel 20: 2-(4-Chlorphenyl)-2-[2-(4-chlorphenoxy)-ethoxy]-1-(2-methyl-imidazol-1-yl)ethylen vom Schmelzpunkt 128 bis 130°C.

Beispiel 21: 2-(4-Chlorphenyl)-2-[2-(2,4-dichlorphenoxy)-ethoxy]-1-(2-methyl-imidazol-1-yl)ethylen vom Schmelzpunkt 132 bis 134°C.

Beispiel 22: 1-(2,4-Dichlorphenyl)-1-(4-chlorphenoxyethoxy)-2-(2-methyl-imidazol-1-yl)butennitrat. Das Produkt zeigt einen nicht ganz scharfen Schmelzpunkt im Bereich von 115 bis 120°C, wobei ein Beginn des Aufschmelzens bereits bei 75°C beobachtet wird. Das Produkt wird daher als Isomerengemisch mit vorwiegendem Anteil an hochschmelzendem Isomer angesehen.

Beispiel 23: 1-(2,4-Dichlorphenyl)-1-(4-chlorphenoxyethoxy)-2-(2-methyl-imidazol-1-yl)butennitrat. Die Substanz wird nach säulenchromatographischer Trennung aus der nach Bei spiel 28 hergestellten Substanz erhalten und zeigt einen ebenfalls etwas unscharfen Schmelzpunkt von 81 bis 92°C, wobei ein erster Schmelzbeginn ab ungefähr 67°C beobachtet wird. Die Substanz wird als Isomerengemisch mit überweigendem Anteil des tieferschmelzenden Isomeren angesehen.

Beispiel 24: 1-(n-Butoxy)-1-(4-nitrophenyl)-2-(2-methylimidazol-1-yl)ethylennitrat vom Schmelzpunkt 119 bis 120°C.

Beispiel 25: 1-(2,4-Dichlorphenyl)-1-(ethoxyethoxy)-2-(imidazol-1-yl)ethylennitrat vom Schmelzpunkt 148 bis 150°C.

Beispiel 26: Z-1-(2,4-Dichlorphenyl)-1-(4-chlorphenoxyethoxy)-2-(imidazol-1-yl)propennitrat vom Schmelzpunkt 141 bis 143°C.

Beispiel 27: 1-(2,4-Dichlorphenyl)-1-(butoxyethoxy)-2-(imidazo-1-yl)ethylennitrat vom Schmelzpunkt 91 bis 92°C.

Beispiel 28: 1-(2,4-Dichlorphenyl)-1-ethoxy-2-(2-phenylimidazol-1-yl)ethylennitrat vom Schmelzpunkt 110 bis 112°C.

Die Ergebnisse der Mikrobiologischen Effektivitätsprüfung der vorstehend genannten Beispiele 17 bis 28 sind in der Tabelle 2 zusammengefaßt. Dabei bedeutet "r", daß Teilresistenzen beobachtet werden.

tabelle 2
MIC (μg/ml)

| | Bakterien | | Pilze | | |
|---|---|---|---|---|---|
| Beispiel | St | Str | C.a. | Tri | Asp |
| 17 | <10 | <10 | | <10 | 25r |
| 18 | r | | r | <10 | |
| 19 | 15 | <10 | | <10 | r |
| 20 | 70r | <10 | r | <10 | <10 |
| 21 | <10 | <10 | | <10 | r |
| 22 | <10 | <10 | r | <10 | 10 |
| 23 | 10 | 10 | | r | |
| 25 | r | | r | <10 | <10 |
| 26 | 10 | | r | 15r | r |
| 27 | 10 | 20 | 25r | <10 | <10 |
| 28 | 10 | r | | <10 | |

**Patentansprüche für den Vertragsstaaten: BE CH DE FR GB IT LU NL SE**

1. Imidazolylvinylether der Formel I

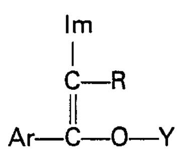

(I)

und deren Säureadditionssalze, wobei in der Formel I

Ar Phenyl, Naphtyl, Thienyl oder Pyridyl bedeutet, wobei diese Ringe einfach oder mehrfach substituiert sein können und die Substituenten unabhängig voneinander Halogen, niederes Alkyl oder Cycloalkyl mit bis zu 6 Kohlenstoffatomen, Trifluormethyl, niederes Alkoxy und niederes Alkylthio mit jeweils 1 bis 6 Kohlenstoffatomen im Alkylteil, Phenyl, Benzyl, Cyano, Nitro oder Amino sein können,

R Wasserstoff, ein unverzweigtes oder verzweigtes Alkyl mit bis zu 6 Kohlenstoffatomen, Phenyl oder ein phenyl-substituiertes Alkyl mit bis zu 6 Kohlenstoffatomen im Alkylteil sein kann, wobei das Phenyl jeweils mit Halogen oder Alkyl oder Alkoxy mit bis zu 6 Kohlenstoffatomen im Alkylteil substituiert sein kann,

Im die 1-H-Imidazol-1-yl-Gruppe bedeutet, die einfach mit Phenyl oder der Nitrogruppe oder einfach, zweifach oder dreifach mit Haloten oder einem Alkyl- oder Alkoxyrest mit 1 bis 4 Alkylkohlenstoffatomen substituiert sein kann, und

Y ein unverzweigtes, verzweigtes oder cyclisches, gesättigtes oder ungesättigtes, eine Doppelbindung aufweisendes Alkyl mit bis zu 6 Kohlenstoffatomen ist, das endständig eine Diethylaminogruppe tragen kann, Phenyl, Naphthyl, Thienyl oder Pyridyl oder ein mit Phenyl, Naphthyl, Pyridyl oder Thienyl substituiertes Alkyl mit 1 bis 6 Kohlenstoffatomen im Alkylteil bedeutet, wobei alle vorstehend genannten aromatischen Substituenten jeweils in der vorstehend für den Rest Ar genannten Weise substituiert sein können und eine direkt an den Ethersauerstoff gebundene Alkylgruppe oder eine direkt an den Ethersauerstoff gebundene Alkylengruppe einmal oder zweimal in der Kette die zweiwertigen Gruppen —O—, —S—, —SO— oder —$SO_2$— aufweisen kann oder eine dieser Gruppen in ω-Stellung zum Ethersauerstoff endständig tragen kann, wobei diese Gruppe dann mit einem der für Ar oben genannten Reste abgesättigt ist, und wobei R nur dann Wasserstoff sein kann, wenn Y nicht mit einer der Gruppen $—CH_2O—$, $—CH_2—S$, $—CH_2SO—$ oder $—CH_2SO_2—$ am Vinylethersauerstoff gebunden ist.

2. Verfahren zur Herstellung neuer Imidazolylvinylether der Formel I

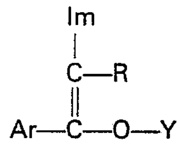

(I)

und deren Säureadditionssalze, wobei in der Formel I

Ar Phenyl, Naphthyl, Thienyl oder Pyridyl bedeutet, wobei diese Ringe einfach oder mehrfach substituiert sein können und die Substituenten unabhängig voneinander Halogen, niederes Alkyl oder Cycloalkyl mit bis zu 6 Kohlenstoffatomen, Trifluormethyl, niederes Alkoxy und niederes Alkylthio mit jeweils 1 bis 6 Kohlenstoffatomen im Alkylteil, Phenyl, Benzyl, Cyano, Nitro oder Amino sein können,

R Wasserstoff, ein unverzweigtes oder verzweigtes Alkyl mit bis zu 6 Kohlenstoffatomen, Phenyl oder ein phenyl-substituiertes Alkyl mit bis zu 6 Kohlenstoffatomen im Alkylteil sein kann, wobei das Phenyl jeweils mit Halogen oder Alkyl oder Alkoxy mit bis zu 6 Kohlenstoffatomen im Alkylteil substituiert sein kann,

Im die 1-H-Imidazol-1-yl-gruppe bedeutet, die einfach mit Phenyl oder der Nitrogruppe oder einfach, zweifach oder dreifach mit Halogen oder einem Alkyl- oder Alkoxyrest mit 1 bis 4 Alkylkohlenstoffatomen substituiert sein kann, und

Y ein unverzweigtes, verzweigtes oder cyclisches, gesättigtes oder ungesättigtes, eine Doppelbindung aufweisendes Alkyl mit bis zu 6 Kohlenstoffatomen ist, das endständig eine Diethylaminogruppe tragen kann, Phenyl, Naphthyl, Thienyl oder Pyridyl oder ein mit Phenyl, Naphthyl, Pyridyl oder Thienyl substituiertes Alkyl mit 1 bis 6 Kohlenstoffatomen im Alkylteil bedeutet, wobei alle vorstehend genannten aromatischen Substituenten jeweils in der vorstehend für den Rest Ar genannten Weise substituiert sein können und eine direkt an den Ethersauerstoff gebundene Alkylgruppe oder eine direkt an den Ethersauerstoff gebundene Alkylengruppe einmal oder zweimal in der Kette die zweiwertigen Gruppen —O—, —S—, —SO— oder $—SO_2—$ aufweisen kann oder eine dieser Gruppen in $\omega$-Stellung zum Ethersauerstoff endständig tragen kann, wobei diese Gruppe dann mit einem der für A oben genannten Reste abgesättigt ist, und wobei R nur dann Wasserstoff sein kann, wenn Y nicht mit einer der Gruppen $—CH_2O—$, $—CH_2S—$, $—CH_2SO—$ oder $—CH_2SO_2—$ am Vinylethersauerstoff gebunden ist.

dadurch gekennzeichnet, daß man ein entsprechendes Keton der Formel III

$$Ar—CO—CHR—Im \qquad (III)'$$

in der Ar, R und Im die vorstehend genannte Bedeutung haben, in Gegenwart von Alkalimetallen, Erdalkalimetallen, deren Hydriden oder Alkoholaten, lithiumorganischen Verbindungen, Natriumamid oder einfach oder zweifach N-substituierten Natriumamiden in einem Lösungsmittel mit einer Verbindung der Formel IV

$$Z—Y \qquad (IV),$$

in der Z eine alkalisch abspaltbare Gruppe ist, umsetzt.

3. Verfahren nach Anspruch 2, dadurch gekennzeichnet, daß in Gegenwart von NaH umgesetzt wird.

4. Verfahren nach einem der Ansprüche 2 oder 3, dadurch gekennzeichnet, daß das Lösungsmittel Hexamethylphosphorsäuretriamid ist.

5. Verfahren nach einem der Ansprüche 2 bis 4, dadurch gekennzeichnet, daß die alkalisch abspaltbare Gruppe Z in der Formel (IV) ein Halogenatom ist.

6. Imidazolylvinylether der Formel I nach Anspruch 1 zur Verwendung beider Bekämpfung von durch Pilze und Bakterien hervorgerufenen Krankheiten bei Menschen, Tieren und Pflanzen.

7. Humanmedizinisches oder veterinärmedizinisches Arzneimittel, enthaltend mindestens einen der Imidazolylvinylether nach Anspruch 1 oder dessen pharmazeutisch unbedenkliches Salz, gegebenenfalls neben anderen üblichen Hilfs-, Träger- und/oder Wirkstoffen.

8. Pflanzenschutzmittel, enthaltend mindestens einen der Imidazolylvinylether nach Anspruch 1 oder dessen unbedenkliches Salz, gegebenenfalls neben anderen üblichen Hilfs-, Träger- und/oder Wirkstoffen.

**Patentansprüche für den Vertragsstaat: AT**

1. Verfahren zur Herstellung neuer Imidazolylvinylether der Formel I

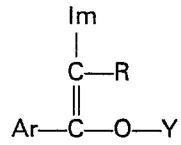

(I)

und deren Säureadditionssalze, wobei in der Formel I

Ar Phenyl, Naphtyl, Thienyl oder Pyridyl bedeutet, wobei diese Ringe einfach oder mehrfach substituiert sein können und die Substituenten unabhängig voneinander Halogen, niederes Alkyl oder Cycloalkyl mit bis zu 6 Kohlenstoffatomen, Trifluormethyl, niederes Alkoxy und niederes Alkylthio mit jeweils 1 bis 6 Kohlenstoffatomen im Alkylteil, Phenyl, Benzyl, Cyano, Nitro oder Amino sein können,

R Wasserstoff, ein unverzweigtes oder verzweigtes Alkyl mit bis zu 6 Kohlenstoffatomen, Phenyl oder ein phenyl-substituiertes Alkyl mit bis zu 6 Kohlenstoffatomen im Alkylteil sein kann, wobei das Phenyl jeweils mit Halogen oder Alkyl oder Alkoxy mit bis zu 6 Kohlenstoffatomen im Alkylteil substituiert sein kann,

Im die 1-H-Imidazol-1-yl-Gruppe bedeutet, die einfach mit Phenyl oder der Nitrogruppe oder einfach, zweifach oder dreifach mit Halogen oder einem Alkyl- oder Alkoxyrest mit 1 bis 4 Alkylkohlenstoffatomen substituiert sein kann, und

Y ein unverzweigtes, verzweigtes oder cyclisches, gesättigtes oder ungesättigtes, eine Doppelbindung aufweisendes Alkyl mit bis zu 6 Kohlenstoffatomen ist, das endständig eine Diethylaminogruppe tragen kann, Phenyl, Naphthyl, Thienyl oder Pyridyl oder ein mit Phenyl, Naphthyl, Pyridyl oder Thienyl substituiertes Alkyl mit 1 bis 6 Kohlenstoffatomen im Alkylteil bedeutet, wobei alle vorstehend genannten aromatischen Substituenten jeweils in der vorstehend für den Rest Ar genannten Weise substituiert sein können und eine direkt an den Ethersauerstoff gebundene Alkylgruppe oder eine direkt an den Ethersauerstoff gebundene Alkylengruppe einmal oder zweimal in der Kette die zweiwertigen Gruppen —O—, —S—, —SO— oder —$SO_2$— aufweisen kann oder eine dieser Gruppen in $\omega$-Stellung zum Ethersauerstoff endständig tragen kann, wobei diese Gruppe dann mit einem der für Ar oben genannten Reste abgesättigt ist, und wobei R nur dann Wasserstoff sein kann, wenn Y nicht mit einer der Gruppen —$CH_2O$—, —$CH_2S$, —$CH_2SO$— oder —$CH_2SO_2$— am Vinylethersauerstoff gebunden ist, dadurch gekennzeichnet, daß man ein entsprechendes Keton der Formel III

$$Ar—CO—CHR—Im \qquad (III),$$

in der Ar, R und Im die vorstehend genannte Bedeutung haben, in Gegenwart einer alkalischen Substanz in einem Lösungsmittel mit einer Verbindung der Formel IV

$$Z—Y \qquad (IV),$$

in der Z eine alkalisch abspaltbare Gruppe ist, umsetzt.

2. Verfahren zur Herstellung neuer Imidazolylvinylether der Formel I

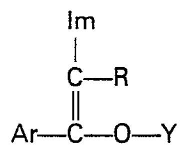

(I)

und deren Säureadditionssalze, wobei in der Formel I

Ar Phenyl, Naphthyl, Thienyl oder Pyridyl bedeutet, wobei diese Ringe einfach oder mehrfach substituiert sein können und die Substituenten unabhängig voneinander Halogen, niederes Alkyl oder Cycloalkyl mit bis zu 6 Kohlenstoffatomen, Trifluormethyl, niederes Alkoxy und niederes Alkylthio mit jeweils 1 bis 6 Kohlenstoffatomen im Alkylteil, Phenyl, Benzyl, Cyano, Nitro oder Amino sein können,

R Wasserstoff, ein unverzweigtes oder verzweigtes Alkyl mit bis zu 6 Kohlenstoffatomen, Phenyl oder ein phenyl-substituiertes Alkyl mit bis zu 6 Kohlenstoffatomen im Alkylteil sein kann, wobei das Phenyl jeweils mit Halogen oder Alkyl oder Alkoxy mit bis zu 6 Kohlenstoffatomen im Alkylteil substituiert sein kann,

Im die 1-H-Imidazol-1-yl-gruppe bedeutet, die einfach mit Phenyl oder der Nitrogruppe oder einfach, zweifach oder dreifach mit Halogen oder einem Alkyl- oder Alkoxyrest mit 1 bis 4 Alkylkohlenstoffatomen substituiert sein kann, und

Y ein unverzweigtes, verzweigtes oder cyclisches, gesättigtes oder ungesättigtes, eine Doppelbindung aufweisendes Alkyl mit bis zu 6 Kohlenstoffatomen ist, das endständig eine

Diethylaminogruppe tragen kann, Phenyl, Naphthyl, Thienyl oder Pyridyl oder ein mit Phenyl, Naphthyl, Pyridyl oder Thienyl substituiertes Alkyl mit 1 bis 6 Kohlenstoffatomen im Alkylteil bedeutet, wobei alle vorstehend genannten aromatischen Substituenten jeweils in der vorstehend für den Rest Ar genannten Weise substituiert sein können und eine direkt an den Ethersauerstoff gebundene Alkylgruppe oder eine direkt an den Ethersauerstoff gebundene Alkylengruppe einmal oder zweimal in der Kette die zweiwertigen Gruppen —O—, —S—, —SO— oder —$SO_2$— aufweisen kann oder eine dieser Gruppen in $\omega$-Stellung zum Ethersauerstoff endständig tragen kann, wobei diese Gruppe dann mit einem der für Ar oben genannten Reste abgesättigt ist, und wobei R nur dann Wasserstoff sein kann, wenn Y nicht mit einer der Gruppen —$CH_2O$—, —$CH_2S$—, —$CH_2SO$— oder —$CH_2SO_2$— am Vinylethersauerstoff gebunden ist,
dadurch gekennzeichnet, daß man ein entsprechendes Keton der Formel III

$$Ar—CO—CHR—Im \qquad (III),$$

in der Ar, R und Im die vorstehend genannte Bedeutung haben, in Gegenwart von Alkalimetallen, Erdalkalimetallen, deren Hydriden oder Alkoholaten, lithiumorganischen Verbindungen, Natriumamid oder einfach oder zweifach N-substituierten Natriumamiden in einem Lösungsmittel mit einer Verbindung der Formel IV

$$Z—Y \qquad (IV),$$

in der Z eine alkalisch abspaltbare Gruppe ist, umsetzt.

3. Verfahren nach Anspruch 2, dadurch gekennzeichnet, daß in Gegenwart von NaH umgesetzt wird.

4. Verfahren nach einem der Ansprüche 2 oder 3, dadurch gekennzeichnet, daß das Lösungsmittel Hexamethylphosphorsäuretriamid ist.

5. Verfahren nach einem der Ansprüche 2 bis 4, dadurch gekennzeichnet, daß die alkalisch abspaltbare Gruppe Z in der Formel (IV) ein Halogenatom ist.

6. Pflanzenschutzmittel, enthaltend mindestens einen der nach Anspruch 1 hergestellten Imidazolylvinylether oder dessen unbedenkliches Salz, gegebenenfalls neben anderen üblichen Hilfs-, Träger- und/oder Wirkstoffen.

7. Verwendung der nach Anspruch 1 hergestellten Imidazolylvinylether der Formel I zur Bekämpfung von durch Pilze und Bakterien hervorgerufenen Krankheiten bei Menschen, Tieren und Pflanzen mittels Fertigpräparaten.

**Claims for contracting states: BE CH DE FR GB IT LU NL SE**

1. Imidazolyl vinyl ether of formula I

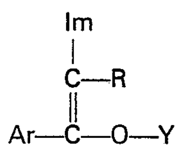

(I)

and acid addition salts thereof, wherein in formula I

Ar is phenyl, naphthyl, thienyl or pyridyl, these rings have optionally one or more substituents and the substituents being independently halogen, lower alkyl or cyclo alkyl containing up to 6 carbon atoms, trifluoromethyl, lower alkoxy and lower alkylthio, each containing 1 to 6 carbon atoms in the alkyl moiety, phenyl, benzyl, cyano, nitro or amino,

R is hydrogen, straight or branched alkyl containing up to 6 carbon atoms, phenyl or by phenyl substituted alkyl containing up to 6 carbon atoms in the alkyl moiety, the phenyl being optionally substituted by halogen or alkyl or alkoxy with up to 6 carbon atoms in the alkyl moiety,

Im is the 1-H-imidazol-1-yl group, which is optionally bearing one phenyl or the nitro group or is optionally substituted by one, two or three substituents selected from halogen or an alkyl or alkoxy group with 1 to 4 carbon atoms in the alkyl moiety, and

Y is straight, branches or cyclic, saturated or unsaturated alkyl with up to 6 carbon atoms containing a double bond, the alkyl group optionally comprising a terminal diethylamino group, phenyl, naphthyl, thienyl or pyridyl or an alkyl with 1 to 6 carbon atoms in the alkyl moiety which is substituted by phenyl, naphthyl, pyridyl or thienyl, wherein all above mentioned aromatic substituents being optionally substituted as cited above for the group Ar and an alkyl group, which is directly attached to the etheral oxygen or an alkylene group which is directly attached to the etheral oxygen can once or twice in the chain comprise the divalent groups —O—, —S—, —SO— or —$SO_2$— or can terminally bear one of these groups in $\omega$-position relative to the etheral oxygen, this group being saturated with one of the groups mentioned above for Ar, and R being hydrogen only in case that Y is not attached to the vinyl ether oxygen by means of one of the groups —$CH_2O$—, —$CH_2S$—, —$CH_2SO$— or —$CH_2SO_2$—

2. A method for preparing new imidazolyl vinyl ethers of formula I

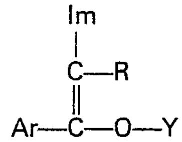

(I)

and acid addition salts thereof, wherein in formula I

Ar is phenyl, naphthyl, thienyl or pyridyl, these rings have optionally one or more substituents and the substituents being independently halogen, lower alkyl or cyclo alkyl containing up to 6 carbon atoms, trifluoromethyl, lower alkoxy and lower alkylthio, each containing 1 to 6 carbon atoms in the alkyl moiety, phenyl, benzyl, cyano, nitro or amino,

R is hydrogen, straight or branched alkyl containing up to 6 carbon atoms, phenyl or by phenyl substituted alkyl containing up to 6 carbon atoms in the alkyl moiety, the phenyl being optionally substituted by halogen or alkyl or alkoxy with up to 6 carbon atoms in the alkyl moiety,

Im is the 1-H-imidazol-1-yl group, which is optionally bearing one phenyl or the nitro group or is optionally substituted by one, two or three substituents selected from halogen or an alkyl or alkoxy group with 1 to 4 carbon atoms in the alkyl moiety, and

Y is straight, branched or cyclic, saturated or unsaturated alkyl with up to 6 carbon atoms containing a double bond, the alkyl group optionally comprising a terminal diethylamino group, phenyl, naphthyl, thienyl or pyridyl or an alkyl with 1 to 6 carbon atoms in the alkyl moiety which is substituted by phenyl, naphthyl, pyridyl or thienyl, wherein all above mentioned aromatic substituents being optionally substituted as cited above for the group Ar and an alkyl group, which is directly attached to the etheral oxygen or an alkylene group which is directly attached to the etheral oxygen can once or twice in the chain comprise the divalent groups —O—, —S—, —SO— or —$SO_2$— or can terminally bear one of these groups in $\omega$-position relative to the etheral oxygen, this group being saturated with one of the groups mentioned above for Ar, and R being hydrogen only in case that Y is not attached to the vinyl ether oxygen by means of one of the groups —$CH_2O$—, —$CH_2S$—, —$CH_2SO$— or —$CH_2SO_2$—

characterized in that an appropriate ketone of formula III

$$Ar—CO—CHR—Im \qquad (III)$$

wherein Ar, R and Im have the above mentioned meaning, is reacted in the presence of alkali metals, alkaline earth metals, hydrides or alcoholates thereof, lithium organic compounds, sodium amide or N-substituted sodium amides bearing one or two substituents at the nitrogen atom in a solvent with a compound of formula IV

$$Z—Y \qquad (IV)$$

wherein Z is a group which is cleavable under alkaline conditions.

3. A method according to claim 2, characterized in that the reaction is carried out in the presence of NaH.

4. A method according to one of claims 2 or 3, characterized in that the solvent is hexamethyl phosphorous triamide.

5. A method according to one of claims 2 to 4, characterized in that the group Z which is cleavable under alkaline conditions in formula IV, is a halogen atom.

6. Imidazolyl vinyl ether of formula I according to claim 1 for use in controlling diseases of humans, animals and plants caused by fungi and bacteria.

7. A drug for humans or animals, containing at least one of the imidazolyl vinyl ethers according to claim 1 of a pharmaceutically acceptable salt thereof, optionally together with other usual adjuvants, carriers and/or active agents.

8. Herbicidal agent, containing at least one of the imidazolyl vinyl ethers of claim 1 or an acceptable salt thereof, optionally together with other usual adjuvants, carriers and/or active agents.

**Claims for contracting state: AT**

1. A method for preparing new imidazolyl vinyl ethers of formula I

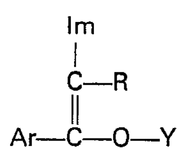

(I)

and acid addition salts thereof, wherein in formula I

Ar is phenyl, naphthyl, thienyl or pyridyl, these rings having optionally one or more substituents and the substituents being independently halogen, lower alkyl or cyclo alkyl containing up to 6 carbon atoms, trifluoromethyl, lower alkoxy and lower alkylthio, each containing 1 to 6 carbon atoms in the alkyl moiety, phenyl, benzyl, cyano, nitro or amino,

R is hydrogen, straight or branched alkyl containing up to 6 carbon atoms, phenyl or by phenyl substituted alkyl containing up to 6 carbon atoms in the alkyl moiety, the phenyl being optionally substituted by halogen or alkyl or alkoxy with up to 6 carbon atoms in the alkyl moiety,

Im is the 1-H-imidazol-1-yl group, which is optionally bearing one phenyl or the nitro group or is optionally substituted by one, two or three substituents selected from halogen or an alkyl or alkoxy group with 1 to 4 carbon atoms in the alkyl moiety, and

Y is straight, branched or cyclic, saturated or unsaturated alkyl with up to 6 carbon atoms containing a double bond, the alkyl group optionally comprising a terminal diethylamino group, phenyl, naphthyl, thienyl or pyridyl or an alkyl with 1 to 6 carbon atoms in the alkyl moiety which is substituted by phenyl, naphthyl, pyridyl or thienyl, wherein all above mentioned aromatic substituents being optionally substituted as cited above for the group.Ar and an alkyl group, which is directly attached to the etheral oxygen or an alkylene group which is directly attached to the etheral oxygen can once or twice in the chain comprise the divalent groups —O—, —S—, —SO— or —$SO_2$— or can terminally bear one of these groups in $\omega$-position relative to the etheral oxygen, this group being saturated with one of the groups mentioned above for Ar, and R being hydrogen only in case that Y is not attached to the vinyl ether oxygen by means of one of the groups —$CH_2O$—, —$CH_2S$—, —$CH_2SO$— or —$CH_2SO_2$—

characterized in that an appropriate ketone of formula III

$$Ar—CO—CHR—Im \qquad (III)$$

wherein Ar, R and Im have the above mentioned meaning, is reacted in the presence of an alkaline substance in a solvent with a compound of formula IV

$$Z—Y \qquad (IV)$$

wherein Z is a group which is cleavable under alkaline conditions.

2. A method for preparing new imidazolyl vinyl ethers of formula I

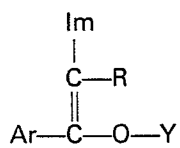

(I)

and acid addition salts thereof, wherein in formula I

Ar is phenyl, naphthyl, thienyl or pyridyl, these rings having optionally one or more substituents and the substituents being independently halogen, lower alkyl or cyclo alkyl containing up to 6 carbon atoms, trifluoromethyl, lower alkoxy and lower alkylthio, each containing 1 to 6 carbon atoms in the alkyl moiety, phenyl, benzyl, cyano, nitro or amino,

R is hydrogen, straight or branched alkyl containing up to 6 carbon atoms, phenyl or by phenyl substituted alkyl containing up to 6 carbon atoms in the alkyl moiety, the phenyl being optionally substituted by halogen or alkyl or alkoxy with up to 6 carbon atoms in the alkyl moiety,

Im is the 1-H-imidazol-1-yl group, which is optionally bearing one phenyl or the nitro group or is optionally substituted by one, two or three substituents selected from halogen or an alkyl or alkoxy group with 1 to 4 carbon atoms in the alkyl moiety, and

Y is straight, branched or cyclic, saturated or unsaturated alkyl with up to 6 carbon atoms containing a double bond, the alkyl group optionally comprising a terminal diethylamino group, phenyl, naphthyl, thienyl or pyridyl or an alkyl with 1 to 6 carbon atoms in the alkyl moiety which is substituted by phenyl, naphthyl, pyridyl or thienyl, wherein all above mentioned aromatic substituents being optionally substituted as cited above for the group Ar and an alkyl group, which is directly attached to the etheral oxygen or an alkylene group which is directly attached to the etheral oxygen can once or twice in the chain comprise the divalent groups —O—, —S—, —SO— or —$SO_2$— or can terminally bear one of these groups in $\omega$-position relative to the etheral oxygen, this group being saturated with one of the groups mentioned above for Ar, and R being hydrogen only in case that Y is not attached to the vinyl ether oxygen by means of one of the groups —$CH_2O$—, —$CH_2S$—, —$CH_2SO$— or —$CH_2SO_2$—

characterized in that an appropriate ketone of formula III

$$Ar—CO—CHR—Im \qquad (III)$$

wherein Ar, R and Im have the above mentioned meaning, is reacted in the presence of alkali metals, alkaline earth metals, hydrides or alcoholates thereof, lithium organic compounds, sodium amide or N-substituted sodium amides bearing one or two substituents at the nitrogen atom in a solvent with a compound of formula IV

$$Z—Y \qquad (IV)$$

wherein Z is a group which is cleavable under alkaline conditions.

3. A method according to claim 2, characterized in that the reaction is carried out in the presence of NaH.

4. A method according to one of claims 2 or 3, characterized in that the solvent is hexamethyl phosphorous triamide.

5. A method according to one of claims 2 to 4, characterized in that the group Z which is cleavable under alkaline conditions in formula IV, is a halogen atom.

6. Crop protective agent comprising at least one of the imidazolyl vinyl ethers prepared in accordance with claim 1 or an acceptable salt thereof, optionally together with other usual adjuvants, carriers and/or active agents.

7. Use of the imidazolyl vinyl ethers of formula I prepared in accordance with claim 1 for controlling diseases of humans, animals and plants caused by fungi and bacteria by means of finished products.

**Revendications pour les états contractants: BE CH DE FR GB IT LU NL SE**

1. Ethers imidazolylvinyliques répondant à la formule (I):

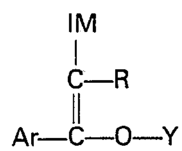

(I)

dans laquelle:

Ar représente un radical phényle, naphtyle, thiényle ou pyridyle éventuellement porteur d'un substituant ou de plusieurs substituants, identiques ou différents, pris dans l'ensemble constitué par les halogènes, les alkyles inférieurs, les cycloalkyles contenant au plus 6 atomes de carbone, le trifluorométhyle, les alcoxy inférieurs et les alkylthio inférieurs contenant chacun de 1 à 6 atomes de carbone dans la partie alkyle, et les radicaux phényle, benzyle, cyano, nitro et amino,

R représente l'hydrogène, un alkyle ramifié ou non ramifié et contenant au plus 6 atomes de carbone, un phényle ou un alkyle porteur d'un phényle et contenant au plus 6 atomes de carbone dans sa partie alkyle, le radical phényle pouvant à chaque fois porter un halogène ou un radical alkyle ou alcoxy contenant au plus 6 atomes de carbone dans sa partie alkyle,

Im représente un radical *1H*-imidazolyle-1 éventuellement porteur d'un seul substituant qui est un radical phényle ou nitro, ou d'un, deux ou trois substituants pris dans l'ensemble constitué par les halogènes et les radicaux alkyles et alcoxy contenant chacun de 1 à 4 atomes de carbone, et

Y représente un alkyle à au plus 6 atomes de carbone, non ramifié, ramifié ou cyclique, saturé ou contenant une double liaison, et portant éventuellement un radical diéthylamino en bout de chaîne, ou représente un radical phényle, naphtyle, thiényle ou pyridyle ou un alkyle en $C_1$—$C_6$ qui porte comme substituant un radical phényle, naphtyle, pyridyle ou thiényle, chacun des substituants aromatiques qui viennent d'être cités pouvant être substitués de la manière indiquée ci-dessus pour le radical Ar, et la chaîne d'un radical alkyle directement relié à l'atome d'oxygène de fonction éther ou d'un radical alkylène directement relié à l'atome d'oxygène de fonction éther pouvant être interrompue une ou deux fois par l'un des radicaux bivalents: —O—, —S—, —SO— et —$SO_2$—, ou pouvant porter l'un de ces radicaux bivalents en bout de chaîne, en position $\omega$ par rapport à l'atome d'oxygène de fonction éther, auquel cas le radical bivalent est saturé par l'un des radicaux mentionnés ci-dessus pour Ar,

avec la condition supplémentaire que R ne peut représenter l'hydrogène que lorsque Y n'est pas relié par un radical —$CH_2O$—, —$CH_2$—S—, —$CH_2SO$— ou —$CH_2SO_2$— à l'atome d'oxygène de l'éther vinylique, ainsi que les sels d'addition qu'ils forment avec des acides.

2. Procédé de préparation de nouveaux éthers imidazolylvinyliques répondant à la formule (I):

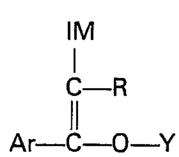

(I)

dans laquelle:

Ar représente un radical phényle, naphtyle, thiényle ou pyridyle éventuellement porteur d'un substituant ou de plusieurs substituants, identiques ou différents, pris dans l'ensemble constitué par les halogènes, les alkyles inférieurs, les cycloalkyles contenant au plus 6 atomes de carbone, le trifluorométhyle, les alcoxy inférieurs et les alkylthio inférieurs contenant chacun de 1 à 6 atomes de carbone dans la partie alkyle, et les radicaux phényle, benzyle, cyano, nitro et amino,

R représente l'hydrogène, un alkyle ramifié ou non ramifié et contenant au plus 6 atomes de carbone, un phényle ou un alkyle porteur d'un phényle et contènant au plus 6 atomes de carbone dans sa partie alkyle, le radical phényle pouvant à chaque fois porter un halogène ou un radical alkyle ou alcoxy contenant au plus 6 atomes de carbone dans sa partie alkyle,

Im représente un radical *1H*-imidazolyle-1 éventuellement porteur d'un seul substituant qui est un radical phényle ou nitro, ou d'un, deux ou trois substituants pris dans l'ensemble constitué par les halogènes et les radicaux alkyles et alcoxy contenant chacun de 1 à 4 atomes de carbone, et

Y représente un alkyle à au plus 6 atomes de carbone, non ramifié, ramifié ou cyclique, saturé ou contenant une double liaison, et portant éventuellement un radical diéthylamino en bout de chaîne, ou représente un radical phényle, naphtyle, thiényle ou pyridyle ou un alkyle en $C_1$—$C_6$ qui porte comme substituant un radical phényle, naphtyle, pyridyle ou thiényle, chacun des substituants aromatiques qui viennent d'être cités pouvant être substitués de la manière indiquée ci-dessus pour le radical Ar, et la chaîne d'un radical alkyle directement relié à l'atome d'oxygène de fonction éther ou d'un radical alkylène directement relié à l'atome d'oxygène de fonction éther pouvant être interrompue une ou deux fois par l'un des radicaux bivalents: —O—, —S—, —SO— et —$SO_2$—, ou pouvant porter l'un de ces radicaux bivalents en bout de chaîne, en position $\omega$ par rapport à l'atome d'oxygène de fonction éther, auquel cas le radical bivalent est saturé par l'un des radicaux mentionnés ci-dessus pour Ar,
avec la condition supplémentaire que R ne peut représenter l'hydrogène que lorsque Y n'est pas relié par un radical —$CH_2O$—, —$CH_2$—S—, —$CH_2SO$— ou —$CH_2SO_2$— à l'atome d'oxygène de l'éther vinylique,
ainsi que des sels d'addition qu'ils forment avec des acides, procédé caractérisé en ce qu'on fait réagir une cétone correspondante de formule (III):

Ar—C—CHR—Im (III)

dans laquelle Ar, R et Im ont les significations précédemment données, en présence de métaux alcalins, de métaux alcalinoterreux, de leurs hydrures ou alcoolates, de composés organiques du lithium, d'amidure de sodium ou d'amidures de sodium monosubstitués ou disubstitués à l'azote, dans un solvant, avec un composé répondant à la formule (IV):

Z—Y (IV)

dans laquelle Z représente un radical éliminable en milieu alcalin.

3. Procédé selon la revendication 2, caractérisé en ce qu'on effectue la réaction en présence de NaH.

4. Procédé selon l'une des revendications 2 et 3, caractérisé en ce que le solvant est l'hexaméthylphosphorotriamide.

5. Procédé selon l'une quelconque des revendications 2 à 4, caractérisé en ce que le radical éliminable en milieu alcalin Z qui est présent dans la formule (IV) est un atome d'halogène.

6. Ethers imidazolylvinyliques de formule (I) selon la revendication 1 devant être utilisés dans la lutte contre des maladies provoquées par des mycètes et des bactéries chez l'homme, des animaux et des plantes.

7. Médicament pour le médecine humaine ou pour la médecine vétérinaire, caractérisé en ce qu'il contient au moins un éther imidazolylvinylique selon la revendication 1 ou l'un de ses sels acceptables du point de vue pharmaceutique, éventuellement associé à d'autres substances actives, excipient et adjuvants usuels.

8. Produits phytosanitaires caractérisés en ce qu'ils contiennent au moins un éther imidazolylvinylique selon la revendication 1 ou un de ses sels inoffensifs, éventuellement associé à d'autres matières actives, supports et adjuvants usuels.

**Revendications pour l'état contractant: AT**

1. Procédé préparation de nouveaux éthers imidazolylvinyliques répondant à la formule (I):

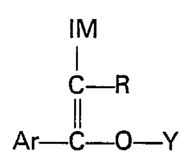

(I)

dans laquelle:

Ar représente un radical phényle, naphtyle, thiényle ou pyridyle éventuellement porteur d'un substituant ou de plusieurs substituants, identiques ou différents, pris dans l'ensemble constitué par les halogènes, les alkyles inférieurs, les cycloalkyles contenant au plus 6 atomes de carbone, le trifluorométhyle, les alcoxy inférieurs et les alkylthio inférieurs contenant chacun de 1 à 6 atomes de carbone dans la partie alkyle, et les radicaux phényle, benzyle, cyano, nitro et amino,

R représente l'hydrogène, un alkyle ramifié ou non ramifié et contenant au plus 6 atomes de carbone, un phényle ou un alkyle porteur d'un phényle et contenant au plus 6 atomes de carbone dans sa partie alkyle, le radical phényle pouvant à chaque fois porter un halogène ou un radical alkyle ou alcoxy contenant au plus 6 atomes de carbone dans sa partie alkyle,

Im représente un radical *1H*-imidazolyle-1 éventuellement porteur d'un seul substituant qui est un radical phényle ou nitro, ou d'un, deux ou trois substituants pris dans l'ensemble constitué par les halogènes et les radicaux alkyles et alcoxy contenant chacun de 1 à 4 atomes de carbone, et

Y représente un alkyle à au plus 6 atomes de carbone, non ramifié, ramifié ou cyclique, saturé ou contenant une double liaison, et portant éventuellement un radical diéthylamino en bout de chaîne, ou représente un radical phényle, naphtyle, thiényle ou pyridyle ou un alkyle en $C_1$—$C_6$ qui porte comme substituant un radical phényle, naphtyle, pyridyle ou thiényle, chacun des substituants aromatiques qui viennent d'être cités pouvant être substitués de la manière indiquée ci-dessus pour le radical Ar, et la chaîne d'un radical alkyle directement relié à l'atome d'oxygène de fonction éther ou d'un radical alkylène directement relié à l'atome d'oxygène de fonction éther pouvant être interrompue une ou deux fois par l'un des radicaux bivalents: —O—, —S—, —SO— et —$SO_2$—, ou pouvant porter l'un de ces radicaux bivalents en bout de chaîne, en position ω par rapport à l'atome d'oxygène de fonction éther, auquel cas ce radical bivalent est saturé par l'un des radicaux mentionnés ci-dessus pour Ar,

avec la condition supplémentaire que R ne peut représenter l'hydrogène que lorsque Y n'est pas relié par un radical —$CH_2O$—, —$CH_2$—S—, —$CH_2SO$— ou —$CH_2SO_2$— à l'atome d'oxygène de l'éther vinylique, ainsi que les sels d'addition qu'ils forment avec des acides caractérisé en ce qu'on fait réagir une cétone correspondante de formule (III):

AR—C—CHR—Im (III)

dans laquelle Ar, R et Im ont les significations précédemment données, en présence de substances alcalines, dans un solvent avec un composé répondant à la formule (IV):

Z—Y (IV)

dans laquelle Z représente un radical éliminable en milieu alcalin.

2. Procédé de préparation de nouveaux éthers imidazolylvinyliques répondant à la formule (I):

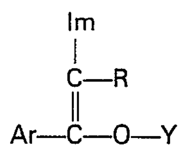

(I)

dans laquelle:

Ar représente un radical phényle, naphtyle, thiényle ou pyridyle éventuellement porteur d'un substituant ou de plusieurs substituants, identiques ou différents, pris dans l'ensemble constitué par les halogènes, les alkyles inférieurs, les cycloalkyles contenant au plus 6 atomes de carbone, le trifluorométhyle, les alcoxy inférieurs et les alkylthio inférieurs contenant chacun de 1 à 6 atomes de carbone dans la partie alkyle, et les radicaux phényle, benzyle, cyano, nitro et amino,

R représente l'hydrogène, un alkyle ramifié ou non ramifié et contenant au plus 6 atomes de carbone, un phényle ou un alkyle porteur d'un phényle et contenant au plus 6 atomes de carbone dans sa partie alkyle, le radical phényle pouvant à chaque fois porter un halogène ou un radical alkyle ou alcoxy contenant au plus 6 atomes de carbone dans sa partie alkyle,

Im représente un radical *1H*-imidazolyle-1 éventuellement porteur d'un seul substituant qui est un radical phényle ou nitro, ou d'un, deux ou trois substituants pris dans l'ensemble constitué par les halogènes et les radicaux alkyles et alcoxy contenant chacun de 1 à 4 atomes de carbone, et

Y représente un alkyle à au plus 6 atomes de carbone, non ramifié, ramifié ou cyclique, saturé ou contenant une double liaison, et portant éventuellement un radical diéthylamino en bout de chaîne, ou représente un radical phényle, naphtyle, thiényle ou pyridyle ou un alkyle en $C_1$—$C_6$ qui porte comme substituant un radical phényle, naphtyle, pyridyle ou thiényle, chacun des substituants aromatiques qui viennent d'être cités pouvant être substitués de la manière indiquée ci-dessus pour le radical Ar, et la chaîne d'un radical alkyle directement relié à l'atome d'oxygène de fonction éther ou d'un radical alkylène directement relié à l'atome d'oxygène de fonction éther pouvant être interrompue une ou deux fois par l'un des radicaux bivalents: —O—, —S—, —SO— et —$SO_2$—, ou pouvant porter l'un de ces

radicaux bivalents en bout de chaîne, en position $\omega$ par rapport à l'atome d'oxygène de fonction éther, auquel cas le radical bivalent est saturé par l'un des radicaux mentionnés ci-dessus pour Ar, avec la condition supplémentaire que R ne peut représenter l'hydrogène que lorsque Y n'est pas relié par un radical $—CH_2O$, $—CH_2—S—$, $—CH_2SO—$ ou $—CH_2SO_2—$ à l'atome d'oxygène de l'éther vinylique,

ainsi que des sels d'addition qu'ils forment avec des acides, procédé caractérisé en ce qu'on fait réagir une cétone correspondante de formule (III):

$$Ar—C—CHR—Im \qquad (III)$$

dans laquelle Ar, R et Im ont les significations précédemment données, en présence de métaux alcalins, de métaux alcalinoterreux, de leurs hydrures ou alcoolates, de composés organiques du lithium, d'amidure de sodium ou d'amidures de sodium monosubstitués ou disubstitués à l'azote, dans un solvant, avec un composé répondant à la formule (IV):

$$Z—Y \qquad (IV)$$

dans laquelle Z représente un radical éliminable en milieu alcalin.

3. Procédé selon la revendication 2, caractérisé en ce qu'on efectue la réaction en présence de NaH.

4. Procédé selon l'une des revendications 2 et 3, caractérisé en ce que le solvant est l'hexaméthylphosphorotriamide.

5. Procédé selon l'une quelconque des revendications 2 à 4, caractérisé en ce que le radical éliminable en milieu alcalin Z qui est présent dans la formule (IV) est un atome d'halogène.

6. Produits phytosanitaires caractérisés en ce qu'ils contiennent au moins un éther imidazolylvinylique selon la revendication 1 ou de ses sels inoffensifs, éventuellement associé à d'autres matières actives, supports et adjuvants usuels.

7. Utilisation des éthers imidazolylvinyliques de formule (I) selon la revendication 1 devant être utilisés dans la lutte contre des maladies provoquées par des mycètes et des bactéries chez l'homme, des animaux et des plantes à l'aide des produits finis.